# EUROPÄISCHE PATENTSCHRIFT

(11) **EP 0 304 886 B1**
(45) Veröffentlichungstag und Bekanntmachung des Hinweises auf die Patenterteilung: **08.12.1993**
(21) Anmeldenummer: 88113779.8
(22) Anmeldetag: 24.08.1988
(51) Int. Cl.: C08G 4/00, C07C 49/84, C07C 45/61, C08F 2/48

(54) **Oligomere Benzilketale und ihre Verwendung als Photoinitiatoren**
Oligomer benzil ketals and their use as photoinitiators
Cétals de benzile oligomères et leur utilisation en tant que photo-initiateurs

(30) Priorität: 27.08.1987 CH 3285/87
(43) Veröffentlichungstag der Anmeldung: 01.03.1989
(73) Patentinhaber: CIBA-GEIGY AG, 4002 Basel (CH)
(72) Erfinder: Hüsler, Rinaldo, Dr., CH-1723 Marly (CH); Kirchmayr, Rudolf, Dr., CH-1723 Marly (CH); Rutsch, Werner, Dr., CH-1700 Fribourg (CH); Rembold, Manfred, Dr., CH-4147 Aesch (CH)
(74) Vertreter: Zumstein, Fritz, Dr.

(56) Entgegenhaltungen:
- EP-A- 0 161 463
- DE-A- 2 602 994

## Beschreibung

Die vorliegende Erfindung betrifft oligomere Benzilketale, ein Verfahren zu ihrer Herstellung und photohärtbare Zusammensetzungen enthaltend solche Benzilketale als Photoinitiatoren.

Die Photopolymerisation ungesättigter Verbindungen hat heute für verschiedene industrielle Sektoren erhebliche Bedeutung erlangt, so z.B. für Lackierungen, für Druckfarben, für die Herstellung von Photoresists und andere Anwendungen in der Elektronik. Hierbei setzt man den zu polymerisierenden Substraten Photoinitiatoren zu, die die Polymerisation und damit die Härtung des Substrates beschleunigen. Diese Initiatoren und ihre Spaltprodukte verbleiben nach der Photopolymerisation im gehärteten Substrat und können an dessen Oberfläche migrieren. Dies kann in gewissen Fällen zu gesundheitlichen Problemen führen, z.B. in Ueberzügen von Lebensmittelverpackungen. Auch können dadurch Geruchsbelästigungen auftreten, z.B. bei Schutzlacken für Bücher oder Schallplattenhüllen.

Um Migration und Verflüchtigung zu unterbinden hat man auch schon die Verwendung polymerer Photoinitiatoren vorgeschlagen. So beschreibt die EP-A-161.463 die Verwendung von Polymeren der Formel 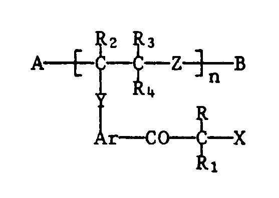
worin X, OH, NH₂, SH oder ein Derivat dieser Gruppen oder Halogen, SCN oder N₃ bedeutet, Ar einen Arylenrest bedeutet und n 2 bis 10⁶ ist. Die Herstellung dieser Produkte erfordert einen relativ grossen Aufwand und ihre Wirkung als Photoinitiatoren ist für viele Anwendungen ungenügend.

In der DE-A-3,534,645 wird die Verwendung acrylisch ungesättigter Photoinitiatoren beschrieben, die bei der Photopolymerisation mit dem Substrat copolymerisieren können. Die Lagerfähigkeit solcher Initiatoren ist jedoch begrenzt.

Es wurde jetzt eine neue Klasse von oligomeren bis polymeren Photoinitiatoren gefunden, die Polyketale von aromatischen 1,2-Diketonen darstellen. Monoketale von aromatischen 1,2-Diketonen (Benzilketale) sind als wirkungsvolle Photoinitiatoren bekannt, z.B. aus den US-A-3,715,293, DE-A-2,232,365 und DE-A-2,337,813, und als solche in industrieller Verwendung. Gegenüber diesen monomeren Benzilketalen haben die erfindungsgemässen Polyketale den Vorteil der geringeren Migrationstendenz, geringeren Extrahierbarkeit, geringeren Geruchsbelästigung und der geringeren Vergilbung des photopolymerisierten Substrates.

Gegenstand der Erfindung sind Polyketale der allgemeinen Formel I und deren Gemische, 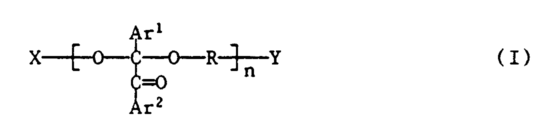
worin X eine Gruppe R¹ oder HO-R- bedeutet,
Y eine Gruppe -OH oder 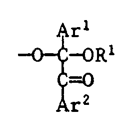
bedeutet,
n ein Wert von 1 bis 30 ist,
Ar¹ und Ar² unabhängig voneinander Phenyl oder durch C₁-C₄-Alkyl, C₁-C₄-Alkoxy oder Halogen substituiertes Phenyl bedeuten,
R ein zweiwertiger Rest ist und zwar entweder
a) ein C₄-C₅₀-Polymethylenrest, der einmal oder mehrmals unterbrochen sein kann durch -O-, -S-, -SO-, -SO₂-, -CO-, -CONH-, -N(R²)-, -O-Si(R³)(R⁴)-O- oder durch einen oder mehrere carbocyclische oder heterocyclische Ringe,
   und der einfach oder mehrfach substituiert sein kann durch C₁-C₁₈-Alkyl, C₃-C₅-Alkenyl, C₅-C₆-Cycloalkyl, Phenyl, Halogen, Cyan, Hydroxy, C₁-C₁₂-Alkoxy, C₁-C₁₂-Alkylthio, Phenoxy, C₂-C₁₂-Alkanoyloxy, Benzoyloxy, C₂-C₅-Alkoxycarbonyl, C₂-C₈-Dialkylamino, Morpholino, Piperidino, Trimethylsiloxy oder durch eine der Gruppen 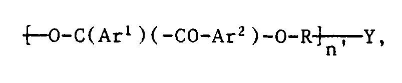 -(C₁-C₄-Alkylen)-R⁶, -O-CH₂CH₂-R⁶, -O-CH₂-CH(CH₃)-R⁶ oder -S-CH₂CH₂-R⁶, worin n′ kleiner als n ist und einen Wert von 0 bis 29 hat,
oder b) ein C₄-C₈-Alkenylen- oder -Alkinylenrest
oder c) ein zweiwertiger cycloaliphatischer Rest mit 6-20 C-Atomen
oder d) ein Rest -CH₂-Z-CH₂- ist,
worin Z ein zweiwertiger cycloaliphatischer, aromatischer oder heterocyclischer Rest mit 4-15 C-Atomen ist,
R¹ C₁-C₄-Alkyl bedeutet,
R² Wasserstoff, C₁-C₄-Alkyl, Cyclohexyl, Phenyl, Chlorphenyl, Tolyl, Benzyl, C₂-C₅-Alkanoyl, Benzoyl, Toluyl oder eine Gruppe -SO₂-R⁵, -CH₂CH₂-R⁶ oder -CH₂-CH(CH₃)-R⁶ bedeutet,
R³ und R⁴ unabhängig voneinander Methyl oder Phenyl bedeutet
R⁵ C₁-C₁₆-Alkyl, Phenyl oder C₇-C₂₀-Alkylphenyl bedeutet und
R⁶ Hydroxy, C₁-C₄-Alkoxy, C₂-C₄-Alkanoyloxy oder 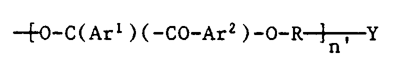
bedeutet.

Ar¹ und Ar² als substituiertes Phenyl können z.B. 4-Tolyl, 2-Tolyl, 4-tert.Butylphenyl, 2-Methoxyphenyl, 4-Methoxyphenyl, 4-Ethoxyphenyl, 4-Isopropoxyphenyl, 4-Chlorphenyl, 4-Bromphenyl oder 4-Fluorphenyl sein. Bevorzugt ist Ar¹ und Ar² Phenyl. R¹ und R² als C₁-C₄-Alkyl können z.B. Methyl, Ethyl, Propyl, Isopropyl, n-Butyl oder Isobutyl sein. Bevorzugt ist R¹ Methyl.

R ist ein durch Entfernung der beiden Hydroxylgruppen aus einem Diol entstehender zweiwertiger Rest. Dafür in Frage kommende Diole werden später aufgeführt. Wenn R ein Polymethylenrest ist, der durch einen oder mehrere carbocyclische oder heterocyclische Ringe unterbrochen ist, so kann der Ring 5- bis 8-gliedrig sein und 3-8 C-Atomen, 1-3 N-Atome, 1-3 O-Atome und/oder 1-2 S-Atome enthalten. Wenn R ein cycloaliphatischer Rest ist, so kann dieser einkernig oder zweikernig sein und 5-20 C-Atome enthalten. Wenn Z ein cycloaliphatischer, aromatischer oder heterocyclischer Rest ist, so kann dieser ein- oder zweikernig sein, 4-15 C-Atome, 1-3 N-Atome, 1-2 O-Atome und/oder 1-2 S-Atome enthalten und die einzelnen Ringe können 5- bis 8-gliedrig sein.

R² als C₂-C₅-Alkanoyl kann z.B. Acetyl, Propionyl, Butyryl, Isobutyryl oder Valeryl sein.

R⁵ als C₁-C₁₆-Alkyl kann z.B. Methyl, Ethyl, Propyl, Butyl, tert.Butyl, Hexyl, Octyl, Decyl, Dodecyl oder Hexadecyl sein. R⁵ als C₇-C₂₀-Alkylphenyl kann z.B. 4-Tolyl, 4-Butylphenyl, 4-Octylphenyl, 4-Dodecylphenyl oder 4-Tetradecylphenyl sein.

Sofern es sich um eine einzelne Verbindung der Formel I handelt, ist der Index n eine ganze Zahl. Meist handelt es sich jedoch um Gemische von Verbindungen mit verschiedenen n und in diesen Gemischen stellt n einen Durchschnittswert dar, der meist keine ganze Zahl ist.

Bevorzugt sind Polyketale der Formel I, worin n 2-20 ist,
Ar¹ und Ar² unabhängig voneinander Phenyl, Tolyl, Chlorphenyl oder Bromphenyl sind,
R ein Polymethylenrest mit 4-30 C-Atomen ist, der einmal oder mehrmals unterbrochen sein kann durch -O-, -S-, -N(R²)- oder durch eine Gruppe 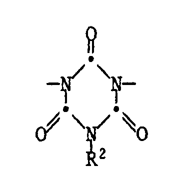
und der ein- oder mehrmals substituiert sein kann durch C₁-C₄-Alkyl, Cyclohexyl, Phenyl, Halogen, C₁-C₄-Alkoxy, Phenoxy, C₇-C₉-Phenylalkyl, C₂-C₈-Dialkylamino, Morpholino oder Piperidino,
R¹ Methyl oder Ethyl ist,
R² Wasserstoff, C₁-C₄-Alkyl, Cyclohexyl, Phenyl, Tolyl, Benzyl, Acetyl, Benzoyl, oder eine Gruppe -CH₂CH₂R⁶ oder -SO₂-R⁵ ist,
R⁵ C₁-C₁₂-Alkyl, Phenyl oder C₇-C₁₈-Alkylphenyl bedeutet,
R⁶ Hydroxy oder 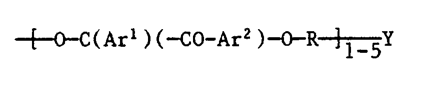
bedeutet und
X und Y die oben gegebene Bedeutung haben.

Besonders bevorzugt sind Polyketale der Formel I, worin n 2-10 ist,
Ar¹ und Ar² Phenyl sind,
R entweder ein Rest -(CH₂)ₘ- mit m = 5-10 ist oder ein Rest -(CH₂CH₂O)ₚCH₂CH₂- mit p = 1-14 ist oder ein Rest -CH₂CH₂-N(R²)-CH₂CH₂-ist,
R¹ Methyl ist, R² C₁-C₄-Alkyl, Cyclohexyl, Phenyl oder Benzyl ist und X und Y die oben gegebene Bedeutung haben, insbesonders Polyketale der Formel I, worin n 2-10 ist, Ar¹ und Ar² Phenyl sind, R ein Rest -(CH₂CH₂O)ₚCH₂CH₂- mit p = 1-10 oder ein Rest -(CH₂)₆- ist und R¹ Methyl ist.

Die erfindungsgemässen Polyketale können hergestellt werden durch eine Transketalisierung von Benzil-dialkylketalen der Formel II mit Diolen der Formel III: 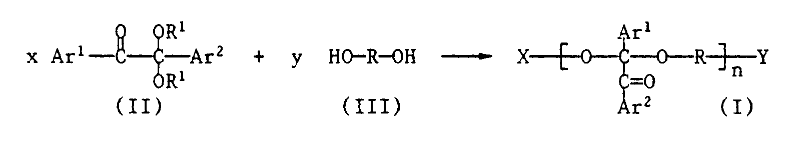
wobei das molare Verhältnis der Komponenten x:y im Bereich von 0,5 bis 2 liegt.
Ist das molare Verhältnis x:y nahe bei 0,5, so entstehen Polyketale mit niedrigem n und Hydroxylendgruppen (X = HO-R-, Y = -OH). Ist das molare Verhältnis x:y 1 oder nahe 1, so entstehen Polyketale, die sowohl Hydroxyl- wie Ketal-Endgruppen haben. Ist das molare Verhältnis x:y nahe 2, so entstehen Polyketale mit vorwiegend Ketal-Endgruppen (X = R¹, Y = -O-C(Ar¹)(COAr²)-OR¹). Man hat es also durch Variation des Molverhältnisses in der Hand, die Eigenschaften der Produkte zu variieren. Auch durch die Reaktionsdauer kann das Molekulargewicht und können damit die Eigenschaften der Produkte variiert werden.

Die als Ausgangsmaterial verwendeten Dialkylketale der Formel II sind bekannte Verbindungen und z.B. in den US-A-3,715,293, DE-A-2,232,365 und DE-A-2,337,813 beschrieben. Benzil-dimethylketal und Benzil-diethylketal sind kommerziell erhältlich.

Die als Ausgangsmaterial benötigten Diole der Formel III sind ebenfalls bekannte Verbindungen. Viele davon sind im Handel erhältlich. Es kann sich dabei auch um technische Gemische handeln. Beispiele für erfindungsgemäss verwendbare Diole der Formel III sind: Butandiol-1,4, Pentandiol-1,5, Hexandiol-1,6, Heptandiol-1,7, Octandiol-1,8, Nonandiol-1,9, Decandiol-1,10, Dodecandiol-1,12, 3-Methylpentandiol-1,5, Pentandiol-1,4, Hexandiol-2,5, 2-trans-Butendiol-1,4, 2-Butindiol-1,4, 3-Hexindiol-2,5, Cyclohexandiol-1,3 und -1,4, Cyclopentandiol-1,3, Cyclooctandiol-1,5 und -1,4, Decahydronaphthalin-1,4 und -1,5, Dicyclohexyl-4,4′-diol, 1,2- und 1,4-Bis(hydroxymethyl)-cyclohexan, 1,3- und 1,4-Bis(hydroxymethyl)-benzol, 1,4-Bis(hydroxymethyl)-2,3,5,6-tetramethylbenzol, 2,5-Bis(hydroxymethyl)-furan, 2,5-Bis(hydroxymethyl)-tetrahydrofuran, 1,4-Bis(2-hydroxymethoxy)-benzol, Di-, Tri- und Tetraethylenglykol, Polyethylenglykol vom Molekulargewicht 200, 300, 400, 600, 1000 oder 1500, Di- und Tripropylenglykol, Polypropylenglykol vom Molekulargewicht 400, 750 oder 1200, Poly(tetramethylenglykol) vom Molekulargewicht 650 oder 1000, 3,8-Dioxadecan-1,10-diol, 4,10-Dioxadecan-2,11-diol, 5-Oxanonan-3,7-diol, Isopropyliden-dicyclohexanol-4,4′, 2,2-Bis[4-(2-hydroxyethoxy)-phenyl]-propan, 2,2-Bis-[4-(2-hydroxypropoxy)-phenyl]-propan, Terephthalsäure-bis(2-hydroxyethylester), Terephthalsäure-bis(2-hydroxyethylamid), 3-Thiapentandiol-1,5, 4-Thiaheptandiol-1,7, 3,6-Dithiaoctandiol-1,8, Diethanolamin, N-Methyldiethanolamin, N-Butyldiethanolamin, N-tert-Butyldiethanolamin, N-Cyclohexyldiethanolamin, N,N-bis(2-hydroxyethyl)-anilin, N,N-Bis(2-hydroxyethyl)-toluidin, N,N-Bis(2-hydroxethyl)-m-chloranilin, N,N′-Bis(2-hydroxyethyl)-1,4-phenylendiamin, N,N′-Bis(2-hydroxyethyl)-piperazin, Bis(2-hydroxypropyl)-amin, Bis(2-hydroxypropyl)-methylamin, N,N′-Bis(2-hydroxyethyl)-ethylendiamin, N,N′-(2-hydroxethyl)-trimethylendiamin, N,N-Bis(2-hydroxyethyl)-acetamid, N,N-Bis(2-hydroxyethyl)-benzamid, N,N-Bis(2-hydroxyethyl)-toluolsulfonamid, N,N-Bis(2-hydroxyethyl)-benzolsulfonamid oder N,N-Bis(2-hydroxyethyl)-menthansulfonamid.

Es können auch Gemische mehrerer Diole verwendet werden oder Gemische von Diolen mit solchen Triolen oder Polyolen, die keine cyclischen Ketale bilden. Beispiele für mitverwendbare Triole und Tetrole sind Tris-(hydroxyalkyl)-benzole, wie z.B. 1,3,5-Tris(hydroxymethyl)-benzol, 1,3,5-Tris(2-hydroxyethyl-benzol oder 1,3,5-Tris(4-hydroxybutyl)-benzol; Tris(hydroxyalkyl)-cyclohexane, wie z.B. 1,3,5-Tris(hydroxymethyl)-cyclohexan, 1,3,5-Tris(3-hydroxypropyl)-cyclohexan oder 1,3,5-Tris(8-hydroxyoctyl)-cyclohexan; 3-(2-Hydroxyethyl)-pentandiol-1,5; 3-(2-Hydroxyethyl)-3-methylpentandiol-1,5; 3,3-Bis(2-hydroxyethyl)pentandiol-1,5; 4,4-Bis(3-hydroxypropyl)-heptandiol-1,5; Tris(2-hydroxyethyl)-isocyanurat; Triethanolamin; Tris(2-hydroxypropyl)-amin oder N,N,N′,N′-Tetrakis(2-hydroxyethyl)-ethylendiamin.

Die Transketalisierung wird katalysiert durch Säuren. Dies können entweder Protonensäuren sein oder Lewis-Säuren. Beispiele hierfür sind HCl, H₂SO₄, H₃PO₄, CH₃SO₃H, CH₃-C₆H₄SO₃H, CF₃COOH, BF₃ oder AlCl₃. Basische Diole oder Triole werden vor der Reaktion mit einer Säure neutralisiert.

Die Reaktion kann in einem inerten Lösungsmittel wie z.B. Benzol, Xylol oder Chlorbenzol, ausgeführt werden, wird jedoch vorzugsweise ohne Lösungsmittel ausgeführt. Der bei der Transketalisierung entstehende Alkohol R¹OH kann abdestilliert werden und zeigt den Fortschritt der Reaktion an.

Die Reaktion wird durch Erwärmen vervollständigt. Vorzugsweise erwärmt man auf 60-130°C, insbesondere auf 70-90°C.
Wenn die Reaktion beendet ist, entfernt man den Katalysator, beispielsweise durch Abdestillieren oder durch Neutralisation mit einer Base. Das so erhaltene Rohprodukt kann für die meisten Zwecke ohne weitere Reinigung verwendet werden. Die Produkte (Polyketale) sind viskos-flüssige Massen oder niedrig schmelzende harzartige Festkörper und sind in organischen Lösungsmitteln gut löslich.

Eine zweite Methode zur Herstellung der Verbindungen der Formel I besteht in der direkten Ketalisierung von aromatischen 1,2-Diketonen der Formel IV mit den Diolen der Formel III: 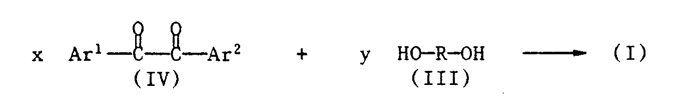
Auch hierbei sind das Molekulargewicht und die Endgruppen der Produkte durch das Molverhältnis x:y in weiten Grenzen variierbar. Als Katalysator werden auch hier Säuren verwendet und man arbeitet vorzugsweise ohne Lösungsmittel. Bevorzugt führt man die Reaktion bei 0 bis 120°C durch, insbesondere bei 0 bis 80°C. Das bei der Reaktion entstehende Wasser kann durch Zusatz eines wasserentziehenden Mittels entfernt werden. Eine besondere Variante dieses Verfahrens ist die Ausführung der Reaktion in Gegenwart mindestens molarer Mengen von Thionylchlorid in Analogie zum Verfahren der DE-B-2,337,813.

Nach den obigen Verfahren werden in der Regel Gemische von Verbindungen der Formel I mit verschiedenem n erhalten. Diese werden in der Regel als solche eingesetzt (siehe unten). Die Einzelkomponenten können aber auch in reiner Form erhalten werden durch übliche physikalische Trennungsmethoden, z.B. durch chromatographische Methoden.

Die Produkte der Formel I sind als Photoinitiatoren für die Photopolymerisation ethylenisch ungesättigter Verbindungen verwendbar. Hierbei kann es sich im einfach oder mehrfach ungesättigte Verbindungen handeln und es kann sich um eine einzelne ungesättigte Verbindung oder um ein Gemisch von ungesättigten Verbindungen handeln.

Beispiele von einfach ungesättigten Verbindungen sind Acrylate oder Methacrylate von einwertigen Alkoholen, Acrylamide und ähnliche Acrylsäurederivate, wie z.B. Methyl-, Ethyl-, Butyl-, Isooctyl- oder Hydroxyethylacrylat, Methyl- oder Ethylmethacrylat, Acrylnitril, Acrylamid, N-Butyl(methacrylamid); sowie Vinyl- und Allylverbindungen wie z.B. Vinylacetat, Vinylstearat, N-Vinylpyrrolidon, Vinylidenchlorid, Vinylbenzol oder Allylacetat.

Beispiele mehrfach ungesättigter Verbindungen sind Acrylate, Methacrylate oder Itaconate von Polyolen wie z.B. Ethylenglykoldiacrylat, Diethylenglykol-dimethacrylat, Triethylenglykol-diacrylat, Butandiol-1,4-diacrylat, Propandiol-1,2-diacrylat, Butandiol-1,3-dimethacrylat, Neopentylglykol-diacrylat, Trimethylolpropan-di(meth)acrylat, Trimethylolethan-di(meth)acrylat, Glycerin-di- und -triacrylat, Pentaerythrit-di-, -tri- und -tetraacrylat oder -methacrylat, Dipentaerythrit-tetra, -penta- und -hexaacrylat oder -methacrylat oder -itaconat, Sorbit-tetraacrylat, Sorbit-hexamethacrylat, Diacrylate oder Dimethacrylate von 1,4-Cyclohexandiol, 1,4-Dimethylolcyclohexan, Bisphenol A, 2,2-Bis(4-hydroxyphenyl)propan von Polyethylenglykolen oder von Oligoestern oder Oligourethanen mit endständigen Hydroxylgruppen. Als mehrfach ungesättigte Monomere können auch Acrylamide verwendet werden wie z.B. Methylenbisacrylamid, Hexamethylen-1,6-bisacrylamid, Diethylentriamin-trismethacrylamid, Bis(methacrylamidopropoxy)ethan oder 2-Acrylamido-ethylacrylat. Beispiele für mehrfach ungesättigte Vinyl- und Allylverbindungen sind Divinylbenzol, Ethylenglykoldivinylether, Diallylphthalat, Allylmethacrylat, Diallylmaleat, Triallylisocyanurat oder Triallylphosphat.

Auch polymere oder oligomere mehrfach ungesättigte Verbindungen lassen sich unter Vernetzung photopolymerisieren wie z.B. ungesättigte Polyester und Copolyester der Maleinsäure und Fumarsäure, (Meth)acrylate von Polyvinylalkohol oder Homo- oder Copolymerisate von Butadien oder Isopren. Weitere verwendbare mehrfach ungesättigte Komponenten sind die Umsetzungsprodukte von Polyepoxiden mit Acryl-oder Methacrylsäuren. Als Polyepoxide werden dabei vorwiegend die im Handel erhältlichen Epoxidharz-Vorprodukte verwendet, die in verschiedenen Molekulargewichten erhältlich sind.

Meist werden zur Photopolymerisation Gemische solcher ungesättigter Verbindungen verwendet, um die Eigenschaften der Polymerisate für den gewünschten Verwendungszweck variieren zu können. Beispiele hierfür sind Gemische von Diacrylaten mit Polyester-acrylaten oder mit Polyurethanacrylaten, Gemische von Mono-, Di- und Triacrylaten, Gemische von ungesättigten Polyestern der Maleinsäure mit Styrol oder andere Gemische von polymer-oligomeren ungesättigten Verbindungen mit Di-, Tri- oder Tetraacrylaten. Die Gemische können aus zwei, drei oder auch mehreren ungesättigten Komponenten bestehen.

Photohärtbare Zusammensetzungen, wie sie für die verschiedenen Zwecke verwendet werden, enthalten meist ausser den photopolymerisierbaren Verbindungen und den Photoinitiatoren eine Reihe sonstiger Zusätze. So ist es vielfach üblich, thermische Inhibitoren zuzusetzen, die vor allem während der Herstellung der Gemische durch Mischen der Komponenten vor einer vorzeitigen Polymerisation schützen sollen. Hierzu werden beispielsweise Hydrochinon, Hydrochinonderivate, p-Methoxyphenol, β-Naphthole oder sterisch gehinderte Phenole wie z.B. 2,6-Di(tert-butyl)-p-kresol verwendet. Weiter können geringe Mengen von UV-Absorbern zugesetzt werden wie z.B. solche vom Benztriazol-, Benzophenon- oder Oxalanilid-Typ. Ebenso lassen sich Lichtschutzmittel vom Typus sterisch gehinderter Amine (HALS) zusetzen.

Zur Erhöhung der Dunkellagerstabilität können Kupferverbindungen wie Kupfernaphthenat, -stearat, oder-octoat, Phosphorverbindungen wie Triphenylphosphin, Tributylphosphin, Triäthylphosphit, Triphenylphosphit oder Tribenzylphosphit, quaternäre Ammoniumverbindungen wie Tetramethylammoniumchlorid oder Trimethyl-benzylammoniumchlorid oder Hydroxylaminderivate, wie z.B. N-Diäthylhydroxylamin, zugesetzt werden.

Die photohärtbaren Zusammensetzungen können auch polymere Bindemittel enthalten, die keine ungesättigten Verbindungen sind. Beispiele hierfür sind Polyacrylate, Celluloseester und -ether, Polyvinylester, Vinylchlorid-polymerisate, Polyamide, Polyester, Polyether oder Styrol-Maleinsäureanhydrid-Copolymere. Weitere übliche Zusatzstoffe sind Pigmente, Farbstoffe, Füllstoffe, Korrosionsinhibitoren, Haftvermittler, Netzmittel oder Verlaufsmittel. Für bestimmte Applikationen können auch Lösungsmittel zugesetzt werden. Vorzugsweise werden jedoch keine Lösungsmittel verwendet.

Weitere übliche Zusätze sind Photosensibilisatoren, welche in bestimmten Wellenlängen absorbieren und die absorbierte Energie an die Initiatoren weitergeben oder selbst als zusätzlicher Initiator fungieren. Beispiele hierfür sind vor allem Thioxanthon-, Anthrachinon- und Cumarinderivate.

Weitere übliche Zusätze sind Beschleuniger vom Amin-Typ, die vor allem in pigmentierten Zubereitungen von Bedeutung sind, da sie als Kettenüberträger wirken. Beispiele hierfür sind N-Methyldiethanolamin, Triethylamin, p-Dimethylaminobenzoesäureethylester oder Michler's Keton.

Gegenstand der Erfindung sind daher auch photohärtbare Zusammensetzungen enthaltend mindestens eine ethylenisch ungesättigte Verbindung und 0,5 bis 20 Gew.-%, insbesondere 1-5 Gew.-%, mindestens einer Verbindung der Formel I.

Die erfindungsgemässen photohärtbaren Zusammensetzungen eignen sich als Beschichtungsmittel für Substrate aller Art, z.B. Holz, Papier, Keramik, Kunststoffe wie Polyester- und Celluloseacetatfilme und Metalle wie Kupfer und Aluminium, bei denen durch Photopolymerisation eine Schutzschicht oder eine Abbildung aufgebracht werden soll.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Insbesondere für den Siebdruck sind UV-härtbare Druckfarben von Bedeutung.

Gut geeignet sind die erfindungsgemässen photohärtbaren Gemische auch zur Herstellung von Druckplatten. Hierbei werden z.B. Gemische von löslichen linearen Polyamiden oder von Styrol-Butadien-Kautschuk mit photopolymerisierbaren Monomeren, beispielsweise Acrylamiden, und einem Photoinitiator verwendet. Filme und Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der Photohärtung ist die Metallbeschichtung, beispielsweise bei der Lackierung von Blechen für Tuben, Dosen oder Flaschenverschlüssen, sowie die Photohärtung von Kunststoffbeschichtungen, beispielsweise von Fussboden- oder Wandbelägen auf PVC-Basis.

Beispiele für die Photohärtung von Papierbeschichtungen sind die farblose Lackierung von Etiketten, Schallplatten-Hüllen oder Buchumschlägen.

Wichtig ist auch die Verwendung der photohärtbaren Zusammensetzungen für Abbildungsverfahren und zur optischen Herstellung von Informationsträgern. Hierbei wird die auf dem Träger aufgebrachte Schicht durch eine Photomaske mit kurzwelligem Licht bestrahlt und die unbelichteten Stellen der Schicht durch Behandlung mit einem Lösungsmittel (= Entwickler) entfernt. Die belichteten Stellen sind vernetzt-polymer und dadurch unlöslich und bleiben auf dem Träger stehen. Bei entsprechender Anfärbung entstehen sichtbare Bilder. Ist der Träger eine metallisierte Schicht, so kann das Metall nach dem Belichten und Entwickeln an den unbelichteten Stellen weggeätzt oder durch Galvanisieren verstärkt werden. Auf diese Weise lassen sich gedruckte Schaltungen und Photoresists herstellen.

Zur Belichtung eignen sich Lichtquellen mit hohem Anteil an kurzwelligem Licht. Hierfür stehen heute entsprechende technische Vorrichtungen und verschiedene Lampenarten zur Verfügung. Beispiele sind Kohlelichtbogenlampen, Xenonlichtbogenlampen, Quecksilberdampflampen, Metall-Halogenlampen, Fluoreszenzlampen, Argonglühlampen oder photographische Flutlichtlampen. Neuerdings werden auch Laserlichtquellen verwendet. Diese lassen sich auch ohne Photomasken einsetzen; der gesteuerte Laserstrahl schreibt dann direkt auf die photohärtbare Schicht. Bei Verwendung als Aussenanstrich ist auch eine Härtung durch Sonnenlicht möglich.

Die folgenden Beispiele erläutern die Herstellung und die Verwendung der neuen Initiatoren. Teile und Prozent beziehen sich darin auf das Gewicht, sofern nichts anderes angegeben wird.

### Herstellungsbeispiele

Ein Gemisch von 153,8 g (0,6 Mol) Benzildimethylketal, 90,1 g (0,6 Mol) Triethylenglykol und 3,9 Toluol-4-sulfonsäure-Monohydrat wird unter Wasserstrahlvakuum (20-30 mbar) auf 75-85°C erwärmt. Bei ca. 63°C ist alles gelöst. Der Reaktionsverlauf wird dünnschichtchromatographisch verfolgt. Die Reaktion wird beendet, wenn im Gemisch weniger als 1 % Benzildimethylketal vorhanden ist oder wenn die Menge an entstehendem Benzil grösser wird als die noch vorhandene Menge an Benzildimethylketal. Dies ist nach 2-3 Stunden der Fall. Die gelbe Lösung wird nun gekühlt und mit 3,8 g einer 30%-igen Lösung von Natriummethylat in Methanol neutralisiert. Dann wird eine Stunde unter Wasserstrahlvakuum auf 60°C erwärmt und das Methanol abdestilliert. Der flüssige Rückstand wird mit 500 ml Toluol verdünnt, mit 10 g Aktivkohle verrührt und über ein Hyflobett filtriert. Das Filtrat wird im Vakuum eingedampft. Es resultieren 208,8 g eines gelben, zähflüssigen Oels (Photoinitiator Nr. 1).

Die Auswertung des 100 Mhz ¹H-NMR-Spektrums des Produktes ergab für die einzelnen Integralbereiche Aromat A (δ 8,3-7,0, 3m, 10H), Diol D (δ 4,0-3,3, m, 12H) und Methoxy M (δ 3,2, 2s, 3H) die Verhältnisse 5:5:1. Diese Werte entsprechend etwa folgender durchschnittlicher Formel: 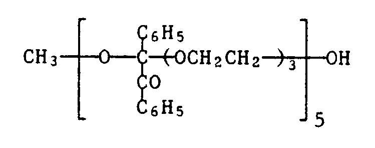
Dies entspricht einer Bruttoformel C₁₀₁H₁₁₄O₂₆ mit einem Molekulargewicht von 1744.

| Elementaranalyse: | | |
|---|---|---|
| ber. | C 69,56 % | H 6,59 % |
| gef. | C 69,54 % | H 6,60 % |

Eine Molekulargewichtsbestimmung durch Gelpermeationschromatographie aus Tetrahydrofuranlösung an Ultrastyragel®-Säulen (Water Ass, Milford, USA) von 1000, 500 und 100 Å Porenwerte ergab ein Zahlenmittel M̅ₙ von 1060 und ein Gewichtsmittel M̅_{w} von 2010.

In analoger Weise wird Benzildimethylketal mit den folgenden Diolen oder Alkoholgemischen im angegebenen Molverhältnis umgesetzt:

| PI Nr. | Aequivalente Diol (pro Aequiv. BDMK) | Verhältnis A:D:M gemäss NMR-Spektrum | Analyse | | M̅ₙ | M̅_{w} |
|---|---|---|---|---|---|---|
| | | | %C | %H | | |
| 2 | 0,8 Triethylenglykol | 20:16:9 | 70,82 | 6,68 | 960 | 2120 |
| 3 | 1,25 Triethylenglykol | 8:10:1 | 67,84 | 6,87 | 1020 | 1840 |
| 4 | 0,55 Triethylenglykol | 7:4:6 | 72,53 | 6,49 | 560 | 1080 |
| 5 | 1 Diethylenglykol | 6:6:1 | 72,15 | 6,29 | 730 | 1970 |
| 6 | 1 Polyethylenglykol 400 | 8:8:1 | 63,39 | 7,51 | 2560 | 9080 |
| 7 | 1 Polyethylenglykol 600 | 5:5:1 | 61,10 | 7,91 | 3200 | 9150 |
| 8 | 1 Polyethylenglykol 1000 | 3:3:1 | 57,71 | 8,27 | 2270 | 5870 |
| 9 | 1 Polytetramethylenglykol 650 | 8:8:1 | 70,03 | 9,40 | 6000 | 21400 |
| 10 | 1 Hexandiol-1,6 | 8:8:1 | 77,22 | 7,31 | 1470 | 3490 |
| 11 | 1 Hydrochinon-bis(2-hydroxyethylether) | 5:5:1 | 73,24 | 5,97 | 1350 | 3170 |
| 12 | 1 Polypropylenglykol 400 | 4:4:3 | 67,19 | 8,52 | 640 | 1530 |
| 13 | 1 Polypropylenglykol 425 | 1:1:1 | 67,05 | 8,69 | 720 | 1520 |
| 14 | 1 Polypropylenglykol 750 | 1:1:1 | 64,90 | 9,22 | 850 | 1940 |
| 15 | 1 Polypropylenglykol 1200 | 1:1:1 | 63,77 | 9,63 | 660 | 2200 |
| 16 | 1 Polyethylenglykol 200 | 4:4:1 | 67,51 | 6,98 | 1190 | 2720 |
| 17 | 1 Polyethylenglykol 300 | 3:3:1 | 64,30 | 7,42 | 1400 | 3060 |
| 18 | 0,8 Hexandiol-1,6 | 20:16:9 | 76,76 | 6,92 | 1010 | 2240 |
| 19 | 1 1,4-Bis(hydroxymethyl)-cyclohexan | 3:3:1 | 77,75 | 7,24 | 686 | 1191 |
| 20 | 0,5 Triethylenglykol und 0,5 Hexandiol-1,6 | 3:3:1 | 73,45 | 7,06 | 1015 | 2389 |
| 21 | 0,8 Triethylenglykol und 0,2 N-Methyldiethanolamin | 5:5:2 | 69,61 | 6,68 | 744 | 1330 |
| 22 | 1 N-Methyldiethanolamin | 3:4:1 | 67,53 | 7,41 | 291 | 318 |
| 23 | 0,80 Triethylenglykol und 0,133 Triethanolamin | 15:14:6 | 69,01 | 6,66 | 507 | 693 |
| 24 | 0,867 Triethylenglykol und 0,133 Triethanolamin | 3:3:1 | 69,67 | 6,77 | 514 | 724 |
| 25 | 0,80 Triethylenglykol und 0,133 Tris(2-hydroxyethyl)-isocyanurat | 15:14:4 | 68,80 | 6,40 | 1098 | 2542 |
| 26 | 1 Tris(2-hydroxyethyl)-isocyanurat | 4:3:2 | 58,71 | 6,75 | 1026 | 1908 |

### Anwendungsbeispiele:

### a) in Polyesterharz

99,5 Teile eines in Styrol gelösten ungesättigten Polyesterharzes (Roskydal®UV502A, Bayer AG) werden mit 0,5 Teilen eines Verlaufshilfsmittels (Byketol®300, Byk-Mallinckrodt) und 2 Teilen des in der Tabelle aufgeführten Polyketal-Photoinitiators unter Erwärmen auf 40-50°C gemischt.

Zur Prüfung der Härtungsgeschwindigkeit (Reaktivität) werden Proben in einer Schichtdicke von 100 µm auf weissen Karton aufgetragen und in einem PPG-Bestrahlungsgerät mit 2 Quecksilber-Mitteldrucklampen à 80 W/cm belichtet. Die Probe wird bei einer Bandgeschwindigkeit von 10 m/min. so oft durch das Gerät durchlaufen gelassen bis die Oberfläche wischfest ist. Die hierfür benötigte Zahl der Durchläufe ist ein Mass für die Reaktivität des Harzes. Je weniger Durchläufe nötig sind, desto reaktiver ist die Probe.

Zur Ermittlung der Durchhärtung werden Proben in einer Schichtdicke von 100 µm auf Glasplatten aufgetragen und in drei Durchläufen bei 10 m/min. im PPG-Bestrahlungsgerät belichtet. Nach 30 Minuten wird die Pendelhärte des gehärteten Films nach der Methode von König (DIN 53 157) bestimmt. Diese ist ein Mass für die Durchhärtung des Filmes.

Der bei der Belichtung der Proben auftretende Geruch wird nach einer 5-stufigen Skala beurteilt. Hierbei bedeutet 1 absolute Geruchsfreiheit und 5 sehr starken unangenehmen Geruch.

| Photoinitiator Nr. | Durchläufe bis Wischfestigkeit | Pendelhärte (sec.) nach 3 Durchläufen | Geruch |
|---|---|---|---|
| 1 | 2 | 132 | 2 |
| 2 | 1 | 126 | 1-2 |
| 3 | 2 | 122 | 1-2 |
| 4 | 1 | 131 | 1-2 |
| 5 | 1 | 136 | 1-2 |
| 6 | 2 | 113 | 2 |
| 7 | 2 | 83 | 2 |
| 10 | 2 | 136 | 2 |
| 11 | 2 | 133 | 2 |
| 12 | 2 | 94 | 2 |
| 13 | 2 | 80 | 2 |
| 16 | 2 | 131 | 2 |
| 17 | 2 | 123 | 2 |
| 18 | 2 | 139 | 2 |
| 19 | 2 | 148 | 2 |
| 20 | 2 | 144 | 3 |
| 21 | 2 | 139 | 3 |
| 22 | 2 | 138 | 3 |
| 24 | 2 | 138 | 3 |
| 25 | 2 | 142 | 3 |
| 26 | 2 | 126 | 2 |

### b) in Acrylatharz

69 Teile eines Epoxiacrylates (Ebecryl®608, UCB), 30 Teile eines oligomeren Triacrylates (OTA 480, UCB) und 1 Teil eines Silicondiacrylates (Ebecryl®350, UCB) werden vermischt und in dieser Mischung werden 2 Teile des in der Tabelle angeführten Polyketal-Photoinitiators unter Erwärmen auf ca. 50°C gelöst.

Weisse Kartons werden mittels einer 6 µm-Spaltrakel mit den Harzproben beschichtet und bei verschiedenen Durchlaufgeschwindigkeiten im PPG-Bestrahlungsgerät mit 2 UV-Lampen à 80 W/cm bestrahlt. Die höchste Geschwindigkeit, bei der in einem Durchgang eine wischfeste Oberfläche erzielt wird, wird als Härtungsgeschwindigkeit angegeben und ist ein Mass für die Reaktivität des Initiators.

Ausserdem werden Glasplatten mittels einer 100 µm-Spaltrakel mit den Proben beschichtet und in einem Durchgang bei einer Geschwindigkeit von 10 m/min im PPG-Bestrahlungsgerät gehärtet. Von diesen Proben wird die Pendelhärte nach König (DIN 53157) bestimmt, was ein Mass für die erfolgte Durchhärtung des Harzes darstellt.

| Photoinitiator Nr. | Härtungsgeschwindigkeit (m/min) | Pendelhärte (sec) |
|---|---|---|
| 2 | 5 | 132 |
| 6 | 4 | 88 |
| 7 | 2,8 | 66 |
| 11 | 4 | 92 |
| 12 | 3,3 | 85 |
| 16 | 5 | 153 |
| 18 | 5 | 146 |
| 19 | 5 | 153 |
| 20 | 5 | 148 |

## Patentansprüche

1. Eine Verbindung oder ein Gemisch von Verbindungen der Formel I, 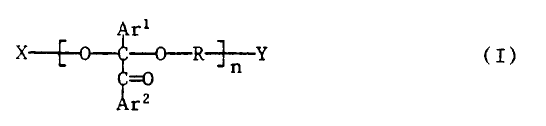 worin X eine Gruppe R¹ oder HO-R- bedeutet,
Y eine Gruppe -OH oder 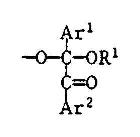 bedeutet,
n ein Wert von 1 bis 30 ist,
Ar¹ und Ar² unabhängig voneinander Phenyl oder durch C₁-C₄-Alkyl, C₁-C₄-Alkoxy oder Halogen substituiertes Phenyl bedeuten,
R ein zweiwertiger Rest ist und zwar entweder
a) ein C₄-C₅₀-Polymethylenrest, der einmal oder mehrmals unterbrochen sein kann durch -O-, -S-, -SO-, -SO₂-, -CO-, -CONH-, -N(R²)-, -O-Si(R³)(R⁴)-O- oder durch einen oder mehrere carbocyclische oder heterocyclische Ringe,
und der einfach oder mehrfach substituiert sein kann durch C₁-C₁₈-Alkyl,
C₃-C₅-Alkenyl, C₅-C₆-Cycloalkyl, Phenyl, Halogen, Cyan, Hydroxy, C₁-C₁₂-Alkoxy, C₁-C₁₂-Alkylthio, Phenoxy, C₂-C₁₂-Alkanoyloxy, Benzoyloxy, C₂-C₅-Alkoxycarbonyl, C₂-C₈-Dialkylamino, Morpholino, Piperidino, Trimethylsiloxy oder durch eine der Gruppen 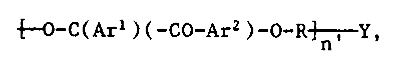 -(C₁-C₄-Alkylen)-R⁶, -O-CH₂CH₂-R⁶, -O-CH₂-CH(CH₃)-R⁶ oder -S-CH₂CH₂-R⁶, worin n′ kleiner als n ist und einen Wert von 0 bis 29 hat,
oder b) ein C₄-C₈-Alkenylen- oder -Alkinylenrest
oder c) ein zweiwertiger cycloaliphatischer Rest mit 6-20 C-Atomen
oder d) ein Rest -CH₂-Z-CH₂- ist,
worin Z ein zweiwertiger cycloaliphatischer, aromatischer oder heterocyclischer Rest mit 4-15 C-Atomen ist,
R¹ C₁-C₄-Alkyl bedeutet,
R² Wasserstoff, C₁-C₄-Alkyl, Cyclohexyl, Phenyl, Chlorphenyl, Tolyl, Benzyl, C₂-C₅-Alkanoyl, Benzoyl, Toluyl oder eine Gruppe -SO₂-R⁵, -CH₂CH₂-R⁶ oder CH₂-CH(CH₃)-R⁶ bedeutet,
R³ und R⁴ unabhängig voneinander Methyl oder Phenyl bedeutet
R⁵ C₁-C₁₆-Alkyl, Phenyl oder C₇-C₂₀-Alkylphenyl bedeutet und R⁶ Hydroxy, C₁-C₄-Alkoxy, C₂-C₄-Alkanoyloxy oder 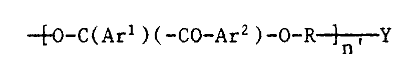 bedeutet.

2. Eine Verbindung oder ein Verbindungsgemisch gemäss Anspruch 1 der Formel I, worin n 2-20 ist,
Ar¹ und Ar² unabhängig voneinander Phenyl, Tolyl, Chlorphenyl oder Bromphenyl sind,
R ein Polymethylenrest mit 4-30 C-Atomen ist, der einmal oder mehrmals unterbrochen sein kann durch -O-, -S-, -N(R²)- oder durch eine Gruppe 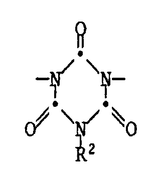 und der ein- oder mehrmal substituiert sein kann durch C₁-C₄-Alkyl, Cyclohexyl, Phenyl, Halogen, C₁-C₄-Alkoxy, Phenoxy, C₇-C₉-Phenylalkyl, C₂-C₈-Dialkylamino, Morpholino oder Piperidino,
R¹ Methyl oder Ethyl ist,
R² Wasserstoff, C₁-C₄-Alkyl, Cyclohexyl, Phenyl, Tolyl, Benzyl, Acetyl, Benzoyl oder eine Gruppe -CH₂CH₂R⁶ oder -SO₂-R⁵ ist,
R⁵ C₁-C₁₂-Alkyl, Phenyl oder C₇-C₁₈-Alkylphenyl bedeutet,
R⁶ Hydroxy oder 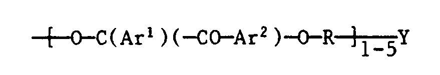 bedeutet und
X and Y die in Anspruch 1 gegebene Bedeutung haben.

3. Eine Verbindung oder ein Verbindungsgemisch gemäss Anspruch 1 der Formel I, worin n 2-10 ist, Ar¹ und Ar² Phenyl sind, R entweder ein Rest -(CH₂)ₘ- mit m = 5-10 ist oder ein Rest -(CH₂CH₂O)ₚCH₂CH₂- mit p = 1-14 ist oder ein Rest -CH₂CH₂-N(R²)-CH₂CH₂- ist, R¹ Methyl ist, R² C₁-C₄-Alkyl, Cyclohexyl, Phenyl oder Benzyl ist und X und Y die in Anspruch 1 gegebene Bedeutung haben.

4. Eine Verbindung oder ein Verbindungsgemisch gemäss Anspruch 1 der Formel I, worin n 2-10 ist, Ar¹ und Ar² Phenyl sind, R ein Rest -(CH₂CH₂O)ₚCH₂CH₂- mit p = 1-10 oder ein Rest -(CH₂)₆- ist, R¹ Methyl ist und X und Y die in Anspruch 1 gegebene Bedeutung haben.

5. Verfahren zur Herstellung von Verbindungen der Formel I und deren Mischungen gemäss Anspruch 1 durch Umsetzung von x Molen einer Verbindung der Formel II 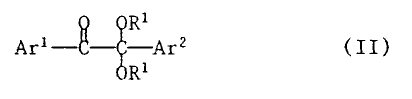 mit y Molen einer oder mehrerer Verbindungen der Formel HO-R-OH in Gegenwart eines sauren Katalysators, wobei Ar¹, Ar², R und R¹ die in Anspruch 1 gegebene Bedeutung haben und wobei das Molverhältnis x:y 0,5 bis 2 beträgt.

6. Verfahren gemäss Anspruch 5, dadurch gekennzeichnet, dass man die Reaktion bei 60-130°C ausführt.

7. Photohärtbare Zusammensetzung, enthaltend mindestens eine ethylenisch ungesättigte Verbindung und 0,5 bis 20 Gew.-% mindestens einer Verbindung der Formel I des Anspruchs 1.

8. Photohärtbare Zusammensetzung gemäss Anspruch 7, enthaltend 1 bis 5 Gew.-% mindestens einer Verbindung des Anspruchs 1.

9. Verwendung von Verbindungen der Formel I des Anspruches 1 als Photoinitiatoren für die Photopolymerisation ethylenisch ungesättigter Verbindungen.

## Claims

1. A compound or a mixture of compounds of the formula I 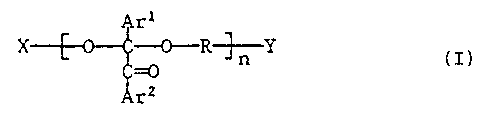 in which X is a group R¹ or HO-R-,
Y is a group -OH or 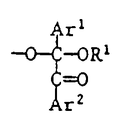 n has a value from 1 to 30,
Ar¹ and Ar² independently of one another are phenyl or phenyl substituted by C₁-C₄alkyl, C₁-C₄alkoxy or halogen, R is a divalent radical and is either a) a C₄-C₅₀polymethylene radical which can be interrupted once or several times by -O-, -S-, -SO-, -SO₂-, -CO-, -CONH-, -N(R²)-, -O-Si(R³)(R⁴)-O- or by one or more carbocyclic or heterocyclic rings, and which can be mono- or polysubstituted by C₁-C₁₈alkyl, C₃-C₅alkenyl, C₅-C₆cycloalkyl, phenyl, halogen, cyano, hydroxyl, C₁-C₁₂alkoxy, C₁-C₁₂alkylthio, phenoxy, C₂-C₁₂alkanoyloxy, benzoyloxy, C₂-C₅alkoxycarbonyl, C₂-C₈dialkylamino, morpholino, piperidino, trimethylsiloxy or by one of the groups [-O-C(Ar²)(-CO-Ar²)-O-R]_{n'}-Y, -(C₁-C₄alkylene)-R⁶, -O-CH₂CH₂-R⁶, -O-CH₂-CH(CH₃)-R⁶ or -S-CH₂CH₂-R⁶, where n' is smaller than n and has a value from 0 to 29, or b) is a C₄-C₈alkenylene or alkynylene radical or c) is a divalent cycloaliphatic radical having 6-20 C atoms or d) is a -CH₂-Z-CH₂- radical, in which Z is a divalent cycloaliphatic, aromatic or heterocyclic radical having 4-15 C atoms, R¹ is C₁-C₄alkyl, R² is hydrogen, C₁-C₄alkyl, cyclohexyl, phenyl, chlorophenyl, tolyl, benzyl, C₂-C₅alkanoyl, benzoyl, toluyl or is a -SO₂-R⁵, -CH₂CH₂-R⁶ or -CH₂-CH(CH₃)-R⁶ group, R³ and R⁴ independently of one another are methyl or phenyl, R⁵ is C₁-C₁₆alkyl, phenyl or C₇-C₂₀alkylphenyl and R⁶ is hydroxyl, C₁-C₄alkoxy, C₂-C₄alkanoyloxy or -[O-C(Ar¹)(-CO-Ar²)-O-R--]_{n'}-Y.

2. A compound or a mixture of compounds according to claim 1 of the formula I, in which n is 2-20,
Ar¹ and Ar² independently of one another are phenyl, tolyl, chlorophenyl or bromophenyl, R is a polymethylene radical having 4-30 C atoms which can be interrupted once or several times by -O-, -S-, -N(R²)- or by a group 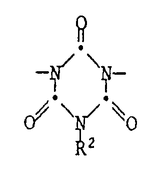 and which can be mono- or polysubstituted by C₁-C₄alkyl, cyclohexyl, phenyl, halogen, C₁-C₄alkoxy, phenoxy, C₇-C₉phenylalkyl, C₂-C₈dialkylamino, morpholino or piperidino, R¹ is methyl or ethyl, R² is hydrogen, C₁-C₄alkyl, cyclohexyl, phenyl, tolyl, benzyl, acetyl, benzoyl or is a group -CH₂CH₂R⁶ or -SO₂-R⁵, R⁵ is C₁-C₁₂alkyl, phenyl or C₇-C₁₈alkylphenyl, R⁶ is hydroxy or -[-O-C(Ar¹)-(-CO-Ar²)-O-R-]₁₋₅Y and X and Y are as defined in claim 1.

3. A compound or a mixture of compounds according to claim 1 of the formula I, in which n is 2-10, Ar¹ and Ar² are phenyl, R is either a -(CH₂)ₘ- radical having an m of 5-10 or is a -(CH₂CH₂O)ₚCH₂CH₂- radical having a p of 1-14 or is a -CH₂CH₂-N(R²)-CH₂CH₂- radical, R¹ is methyl, R² is C₁-C₄alkyl, cyclohexyl, phenyl or benzyl and X and Y are as defined in claim 1.

4. A compound or a mixture of compounds according to claim 1 of the formula I, in which n is 2-10, Ar¹ and Ar² are phenyl, R is a -(CH₂CH₂O)ₚ-CH₂CH₂- radical having a p of 1-10 or is a -(CH₂)₆- radical, R¹ is methyl and X and Y are as defined in claim 1.

5. A process for the preparation of compounds of the formula I and mixtures thereof according to claim 1 by reaction of x moles of a compound of the formula II 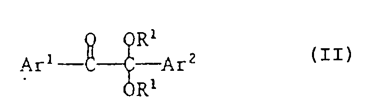 with y moles of one or more compounds of the formula HO-R-OH in the presence of an acid catalyst, Ar¹, Ar², R and R¹ being as defined in claim 1 and the molar ratio x:y being 0.5 to 2.

6. A process according to claim 5, which comprises carrying out the reaction at 60-130°C.

7. A photocurable composition containing at least one ethylenically unsaturated compound and at least 0.5 to 20% by weight of a compound of the formula I of claim 1.

8. A photocurable composition according to claim 7, containing at least 1 to 5% by weight of a compound of claim 1.

9. Use of compounds of the formula I of claim 1 as photoinitiators for the photopolymerization of ethylenically unsaturated compounds.

## Revendications

1. Composé ou mélange de composés répondant à la formule I : 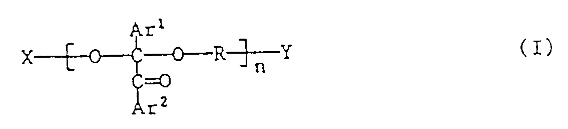 où X signifie un groupe R¹ ou HO-R-,
Y signifie un groupe -OH ou 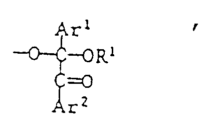 n est une valeur de 1 à 30,
Ar¹ et Ar² représentent chacun, indépendamment l'un de l'autre, un phényle ou un phényle substitué par un alkyle en C₁-C₄, un alcoxy en C₁-C₄ ou un halogène,
R est un radical bivalent à savoir, soit
a) un radical polyméthylène en C₄-C₅₀ qui peut être interrompu une ou plusieurs fois par -O-, -S-, -SO-, -SO₂-, -CO-, -CONH-, -N(R²)-, -O-Si(R³)(R⁴)-O- ou par un ou plusieurs cycles carbocycliques ou hétérocycliques,
et qui peut être mono- ou polysubstitué par un alkyle en C₁-C₁₈, un alcènyle en C₃-C₅, un cycloalkyle en C₅-C₆, un phényle, un halogène, un cyanogène, un hydroxy, un alcoxy en C₁-C₁₂, un alkylthio en C₁-C₁₂, un phénoxy, un alcanoyloxy en C₂-C₁₂, un benzoyloxy, un alcoxycarbonyle en C₂-C₅, un dialkylamino en C₂-C₈, un morpholino, un pipéridino, un triméthylsiloxy ou par un des groupements [-O-C(Ar¹)(-CO-Ar²)-O-R]_{n'}-Y, -(alkylène en C₁-C₄)-R⁶, -O-CH₂CH₂-R⁶, -O-CH₂-CH(CH₃)-R⁶ ou -S-CH₂CH₂-R⁶ où n' est inférieur à n et a une valeur de 0 à 29, soit
b) un radical alcènylène ou un alcynylene en C4-C8 soit
c) un radical cycloaliphatique bivalent avec un nombre de 6 à 20 atomes de C, soit
d) un radical -CH₂-Z-CH₂-,
où Z est un radical bivalent cycloaliphatique, aromatique ou hétérocyclique comportant de 4 à 15 atomes de C,
R¹ signifie un alkyle en C₁-C₄,
R² signifie un hydrogène, un alkyle en C₁-C₄, un cyclohexyle, un phényle, un chlorophényle, un tolyle, un benzyle, un alcanoyle en C₂ - C₅, un benzoyle, un toluyle ou un groupement -SO₂-R⁵, -CH₂CH₂-R⁶ ou CH₂-CH(CH₃)-R⁶,
R³ et R⁴ signifient chacun, indépendamment l'un de l'autre, un méthyle ou un phényle,
R⁵ signifie un alkyle en C₁-C₁₆, un phényle ou un alkylphényle en C₇-C₂₀ et R⁶ signifie un hydroxy, un alcoxy en C₁-C₄, un alcanoyloxy en C₂-C₄ ou un -[O-C(Ar¹)(-CO-Ar²)-O-R-]_{n'}-Y.

2. Composé ou mélange de composés selon la revendication 1, répondant à la formule I où n est une valeur de 2 à 20,
Ar¹ et Ar², sont chacun, indépendamment l'un de l'autre, un phényle, un tolyle, un chlorophényle ou un bromophényle,
R est un radical polyméthylène comportant de 4 à 30 atomes de C, qui peut être une ou plusieurs fois interrompu par -O-, -S-, -N(R²)- ou par un groupement 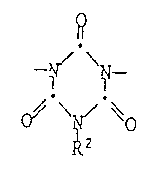 et qui peut être mono- ou polysubstitué par un alkyle en C₁-C₄, un cyclohexyle, un phényle, un halogène, un alcoxyle en C₁-C₄, un phénoxy, un phénylalkyle en C₇-C₉, un dialkylamino en C₂-C₈, un morpholino ou un pipéridino,
R¹ est un méthyle ou un éthyle,
R² est l'hydrogène, un alkyle en C₁-C₄, un cyclohexyle, un phényle, un tolyle, un benzyle, un acétyle, un benzoyle ou un groupement -CH₂CH₂R⁶ ou -SO₂-R⁵,
R⁵ signifie un alkyle en C₁-C₁₂, un phényle ou un alkylphényle en C₇-C₁₈,
R ⁶ signifie un hydroxy ou 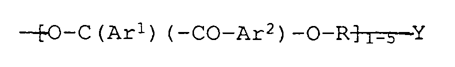 et
X et Y ont la signification donnée dans la revendication 1.

3. Composé ou mélange de composés selon la revendication 1, répondant à la formule I, où n est une valeur de 2 à 10, Ar¹ et Ar² sont un phényle, R est soit un radical -(CH₂)ₘ-avec m = 5-10 ou un radical -(CH₂CH₂O)ₚCH₂CH₂- avec p = 1-14 ou un radical -CH₂CH₂-N(R²)-CH₂CH₂-, R¹ est un méthyle, R² est un alkyle en C₁-C₄, un cyclohexyle, un phényle ou un benzyle et X et Y ont la signification donnée dans la revendication 1.

4. Composé ou mélange de composés selon la revendication 1, répondant à la formule I où n est une valeur de 2 à 10, Ar¹ et Ar² sont un phényle, R est un radical -(CH₂CH₂O)ₚCH₂CH₂- avec p = 1-10 ou un radical -(CH₂)₆-, R¹ est un méthyle et X et Y ont la signification donnée dans la revendication 1.

5. Procédé de préparation de composés répondant à la formule I et de leurs mélanges selon la revendication 1, par réaction de x moles d'un composé répondant à la formule II : 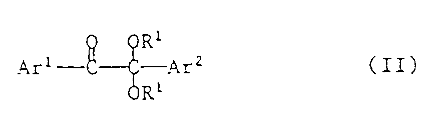 avec y moles d'un ou plusieurs composés de formule HO-R-OH, en présence d'un catalyseur acide, Ar¹, Ar², R et R¹ ayant la signification donnée dans la revendication 1 et le rapport molaire x:y étant de 0,5 à 2.

6. Procédé selon la revendication 5, caractérisé en ce qu'on réalise la réaction à 60-130°C.

7. Composition photodurcissable contenant au moins un composé à insaturation éthylénique et ayant une teneur de 0,5 à 20% en poids en au moins un composé répondant à la formule I de la revendication 1.

8. Composition photodurcissable selon la revendication 7, ayant une teneur de 1 à 5% en poids en un composé selon la revendication 1.

9. Utilisation des composés répondant à la formule I de la revendication 1, comme photo-amorceurs ou photo-initiateurs pour la photopolymérisation de composés à insaturation éthylènique.
